# EUROPEAN PATENT SPECIFICATION

(11) **EP 3 266 468 B1**
(45) Date of publication and mention of the grant of the patent: **14.10.2020**
(21) Application number: 16758935.7
(22) Date of filing: 02.03.2016
(51) Int. Cl.: A61K 9/08, A61K 9/00, A61K 47/32, A61K 31/80, A61P 27/02, A61P 27/04

(54) **EYEDROPS**
AUGENTROPFEN
GOUTTES OCULAIRES

(30) Priority: 03.03.2015 JP 2015041214
(43) Date of publication of application: 10.01.2018
(73) Proprietor: NOF Corporation, Shibuya-ku Tokyo 150-6019 (JP)
(72) Inventor: SAKURAI, Shunsuke, Kawasaki-shi Kanagawa 210-0865 (JP); MIYAMOTO, Koji, Kawasaki-shi Kanagawa 210-0865 (JP); SHIMAMURA, Yoshihisa, Kawasaki-shi Kanagawa 210-0865 (JP); TAKAHASHI, Masatomo, Kawasaki-shi Kanagawa 210-0865 (JP); MATSUOKA, Yosuke, Kawasaki-shi Kanagawa 210-0865 (JP); YAMAMOTO, Nobuyuki, Kawasaki-shi Kanagawa 210-0865 (JP)
(74) Representative: Mewburn Ellis LLP
(86) International application number: PCT/JP2016/056358
(87) International publication number: WO 2016/140242

(56) References cited:
- EP-A1- 2 821 841
- WO-A1-02/15911
- JP-A- 2012 088 524
- EFRON NATHAN ET AL: "Lid wiper epitheliopathy", PROGRESS IN RETINAL AND EYE RESEARCH, OXFORD, GB, vol. 53, 14 April 2016 (2016-04-14), pages 140-174, XP029617310, ISSN: 1350-9462, DOI: 10.1016/J.PRETEYERES.2016.04.004
- SPRINGS, C.L.: 'Novel hydroxypropyl-guar gellable lubricant eye drops for treatment of dry eye' ADVANCES IN THERAPY vol. 27, no. 10, 2010, pages 681 - 690, XP009505660

## Description

### Technical Field

The present invention relates to an aqueous ophthalmic solution for use in the treatment or prevention of lid wiper epitheliopathy.

The present application claims priority from Japanese Patent Application No. 2015-041214

### Background Art

It is said that the number of dry eye patients is increasing because of an increasing number of contact lens wearers and an increase of visual display terminal (VDT) work under air conditioning with an air conditioner or the like. The term "dry eye" is generally recognized as referring literally to an eye being dry, but academically has a clearer definition given in a 2007 report (Non Patent Literature 1). According to this definition of "dry eye", one is diagnosed as having dry eye only when symptoms such as 1) a subjective symptom, 2) a lacrimal fluid disturbance, and 3) conjunctivocorneal epitheliopathy are found. In particular, for lacrimal fluid, there is an established quantitative evaluation method, and many clinical researches have been conducted (Non Patent Literature 2 and Non Patent Literature 3).

In recent years, it has been reported that, among patients who go to hospital complaining of subjective symptoms of dry eye, there are non-dry eye cases in which no disturbance is found in lacrimal function (Non Patent Literature 4). Such case was first reported by Korb et al. in 2002, and was named lid-wiper epitheliopathy (LWE) (Non Patent Literature 5 and Non Patent Literature 6). Korb et al. named a region from the subtarsal sulcus to the mucocutaneous junction lid-wiper because there has been no anatomical name for this portion and this portion moves like wiping an ocular surface, and gave the case the name LWE because the case was epitheliopathy in this portion (Non Patent Literature 5). LWE is recognized as epitheliopathy in upper and lower eyelid marginal conjunctiva (Non Patent Literature 5).

In the LWE, shedding and degeneration of the cortical epithelium of the eyelid marginal conjunctiva are considered to be caused by an increase in friction between the eyelid marginal conjunctiva and the ocular surface due to blinking (Non Patent Literature 6). For treatment of LWE for a contact lens wearer, it is presumably preferred to stop the wearer from wearing contact lenses and perform the treatment by administering an artificial tear. In addition, for treatment of LWE for a non-contact lens wearer, it is presumably effective to reduce friction on the ocular surface with a hyaluronic acid ophthalmic solution or an ophthalmic ointment.

As described above, for treatment of the relatively new disease LWE, it is essential to improve lubricity on a corneal surface. Accordingly, research and development of pharmaceutical compositions, active pharmaceutical ingredients, and pharmaceutical additives that improve the lubricity on the corneal surface have heretofore been conducted.

For example, in Patent Literature 1, there is a disclosure of a method of improving lubricity by blending jojoba wax, sperm oil, orange roughy oil, or the like. However, when the method is applied to an ophthalmic solution that is mostly water, it has been difficult to obtain a satisfactory composition.

In Patent Literature 2, there is a disclosure of a "contact lens wetting solution containing a copolymer obtained by polymerizing a monomer composition containing 2-(meth)acryloyloxyethyl phosphorylcholine and an alkyl (meth) acrylate, in which a carbon number of an alkyl group in the alkyl (meth)acrylate, and a molar fraction of a constituent unit derived from the alkyl (meth) acrylate with respect to a total amount of constituent units derived from the 2-(meth)acryloyloxyethyl phosphorylcholine and the alkyl (meth)acrylate are specifically set, and in which a molecular weight of the copolymer is controlled. " However, a copolymer contained in an ophthalmic solution of the present invention is clearly different in structure from the copolymer disclosed in Patent Literature 2.

In Patent Literature 3, wettability of a soft contact lens is improved and a contact lens surface is made moist to improve a sense of use. However, a lubricity-improving effect provided by the method of Patent Literature 3 is insufficient.

In Patent Literature 4, there is a disclosure of a composition capable of significantly improving lubricity, which relates to a contact lens care preparation, but there is no mention of an example of an ophthalmic solution or lid wiper epitheliopathy.

In Patent Literature 5, there is disclosed a contact lens solution that may be used to impart surface lubricity and amebic adhesion inhibitory effects to a surface of a contact lens. The solution comprises 0.01 to 2 weight/volume% of a polymer having monomer units comprising a phosphorylcholine-like group, particularly (meth)acrylamide derivative units, and monomer units with a hydrophilic group.

### Citation List

### Patent Literature

[PTL 1] JP 2007-528897 A
[PTL 2] WO 2002/015911 A1
[PTL 3] JP 2011-136923 A
[PTL 4] JP 2012-88524 A
[PTL 5] EP 2821841 A1

### Non Patent Literature

[NPL 1] 2007 Report of the International Dry Eye Workshop (DEWS), 2007, Ocul. Surf., 5, 65-204.
[NPL 2] T. Goto et al., 2003, Am. J. Ophthalmol, 135, 607-612.
[NPL 3] K. Maruyama et al., 2004, IOVS, 45, 2563-2568.
[NPL 4] B. Yenaid et al., 2010, Eye & Contact Lens, 36, 140-143.
[NPL 5] D.R. Korb et al., 2002, CLAO J, 28, 211-216.
[NPL 6] D.R. Korb et al., 2005, Eye & Contact Lens, 31, 2-8.

### Summary of Invention

### Technical Problem

None of the related-art methods provides sufficient lubricity, and under the circumstances, sufficient lubricity has not been obtained for a corneal surface or a soft contact lens surface .

That is, under the circumstances, there has not been obtained a satisfactory ophthalmic solution for imparting sufficient lubricity to a corneal surface or a soft contact lens surface to treat or prevent lid wiper epitheliopathy.

In view of the foregoing, an object of the present invention is to provide an ophthalmic solution for use in the treatment or prevention of lidwiper epitheliopathy, which is capable of imparting sufficient lubricity to a corneal surface or a soft contact lens surface.

### Solution to Problem

The inventors of the present invention have made extensive investigations in order to achieve the above-mentioned object, and as a result, have found that the object can be achieved by the use of an ophthalmic aqueous solution containing, at a specific ratio, a copolymer having a structure having three kinds of different constituent units at a specific ratio. Thus, the inventors have completed the present invention.

That is, the present invention is as described below.
1. An ophthalmic aqueous solution for use in treatment or prevention of lid wiper epitheliopathy, the solution including 0.01 W/V% to 2.0 W/V% of a copolymer (P) having constituent units represented by the formula (1a) to the formula (1c), a ratio nₐ:n_{b}:n_{c} of the constituent units of 100:from 10 to 400:from 2 to 50, and a weight-average molecular weight of from 5,000 to 2,000,000: 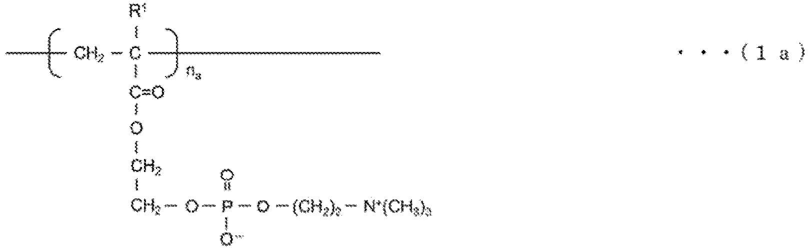 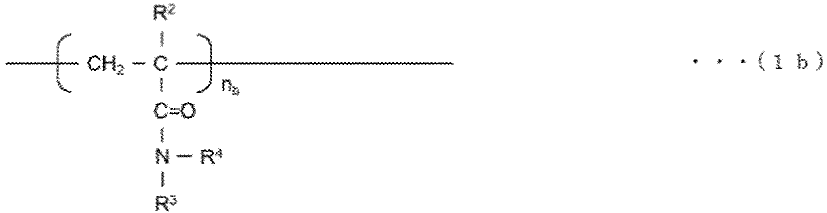 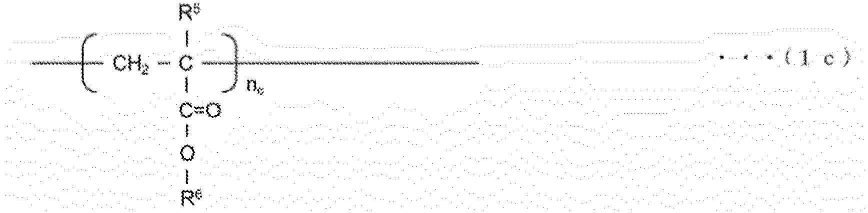 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.
2. An ophthalmic aqueous solution for use according to the above-mentioned item 1, in which the (1a) includes a constituent unit obtained from 2-(methacryloyloxy)ethyl-2'-(trimethylammonio)ethyl phosphate, the (1b) includes a constituent unit obtained from N,N-dimethyl(meth)acrylamide, and the (1c) includes a constituent unit obtained from stearyl (meth)acrylate.
3. An ophthalmic aqueous solution for use according to the above-mentioned item 1, in which the (1a) includes a constituent unit obtained from 2-(methacryloyloxy)ethyl-2'-(trimethylammonio)ethyl phosphate, the (1b) includes a constituent unit obtained from N,N-dimethyl(meth)acrylamide, and the (1c) includes a constituent unit obtained from lauryl (meth)acrylate.
4. An ophthalmic aqueous solution for use according to any one of the above-mentioned items 1 to 3, in which the ophthalmic solution is for use in a method of treatment of a subject, wherein the subject is a soft contact lens wearer.
5. An ophthalmic aqueous solution for use in treatment or prevention of wiper lid epitheliopathy may include use of a copolymer (P) having constituent units represented by the formula (1a) to the formula (1c), a ratio nₐ: n_{b}:n_{c} of the constituent units of 100: from 10 to 400:from 2 to 50, and a weight-average molecular weight of from 5,000 to 2,000,000: 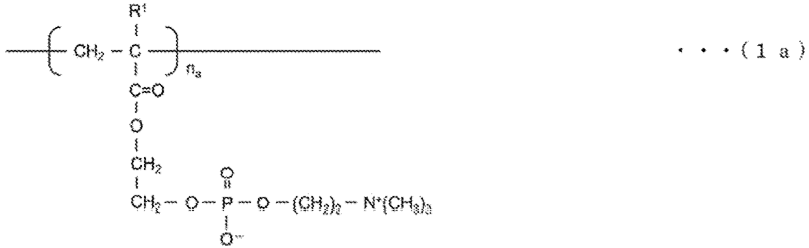 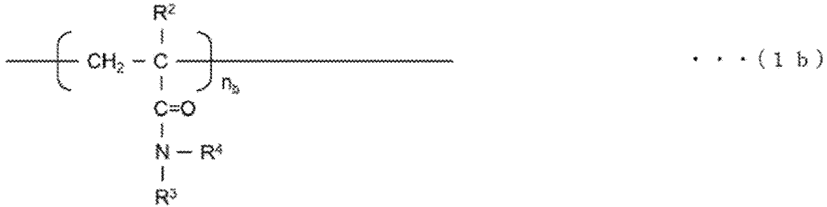 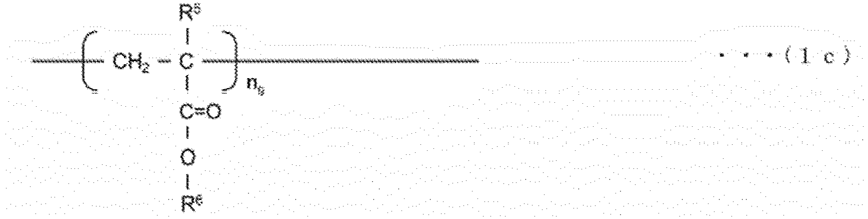 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms according to item 1. Item 5 is not part of the claims.

### Advantageous Effects of Invention

The ophthalmic aqueous solution for use in treatment or prevention of lid wiper epitheliopathy ,and the ophthalmic solution for use in a method of treatment of a subject wherein the subject is a soft contact lens wearer are capable of imparting sufficient lubricity to a corneal surface and a soft contact lens surface, and are useful for treatment or prevention of lid wiper epitheliopathy.

### Description of Embodiments

Now, the present invention is described in more detail.

A copolymer (P) to be used in an ophthalmic aqueous solution (referred to hereinafter as the ophthalmic solution) of the present invention has the following three constituent units (1) to (3), and has a ratio (molar ratio) nₐ:n_{b}:n_{c} of the constituent units of 100:from 10 to 400:from 2 to 50.

### <(1) PC Constituent Unit>

The copolymer (P) to be used in the ophthalmic solution of the present invention has a constituent unit represented by the following formula (1a) (hereinafter abbreviated as "PC constituent unit").

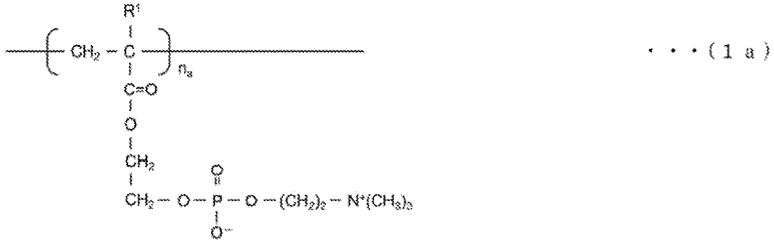

In the formula (1a), R¹ represents a hydrogen atom or a methyl group.

The PC constituent unit in the copolymer (P) is introduced in order to impart hydrophilicity and hydrous gel-forming ability to the copolymer (P) to enhance lubricity.

The PC constituent unit in the copolymer (P) is obtained from a phosphorylcholine-like group-containing monomer represented by the following formula (2) (hereinafter referred to as "PC monomer") to be used at the time of polymerization of the copolymer (P).

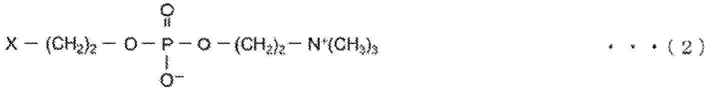

In the formula (2), X represents a monovalent organic group having a polymerizable functional group having an unsaturated bond.

As the PC monomer, from the viewpoint of availability, for example, 2-((meth)acryloyloxy)ethyl-2'-(trimethylammonio)ethyl phosphate is preferred, and 2-(methacryloyloxy)ethyl-2'-(trimethylammonio)ethyl phosphate represented by the following formula (3) (hereinafter referred to as MPC) is more preferred.

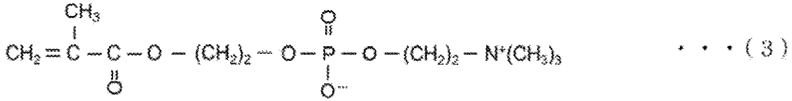

The PC monomer may be produced by a known method. The PC monomer may be produced by, for example, a method disclosed in JP 54-63025 A, which involves allowing a compound, which is obtained by allowing a hydroxy group-containing polymerizable monomer and 2-bromoethylphosphoryl dichloride to react with each other in the presence of a tertiary base, and a tertiary amine to react with each other, or a method disclosed in JP 58-154591 A or the like, which involves obtaining a cyclic compound by a reaction between a hydroxy group-containing polymerizable monomer and a cyclic phosphorus compound, and then subjecting the cyclic compound to a ring-opening reaction with a tertiary amine.

### <(2) Amide Constituent Unit>

The copolymer (P) to be used in the ophthalmic solution of the present invention has a constituent unit represented by the following formula (1b) (hereinafter abbreviated as "amide constituent unit").

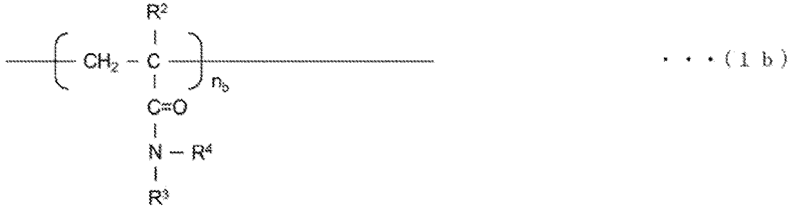

In the formula (1b), R² represents a hydrogen atom or a methyl group, and R³ and R⁴ each independently represent a hydrogen atom, a methyl group, or an ethyl group, or are bonded to each other to represent a morpholino group.

The amide constituent unit in the copolymer (P) is introduced in order to increase the molecular weight of the copolymer (P) to enhance adherence to a soft contact lens.

With regard to the ratio of the amide constituent unit in the copolymer (P), the ratio of the number of moles n_{b} to the number of moles nₐ of the PC constituent unit being defined as 100, i.e., n_{b}/nₐ is from 10/100 to 400/100, preferably from 30/100 to 250/100. When n_{b} is excessively large, there is a risk in that aseptic filtration to be required in the production of the ophthalmic solution may be difficult. When n_{b} is excessively small, a lubricity-improving effect cannot be expected.

The amide constituent unit in the copolymer (P) is obtained from a monomer represented by the following formula (1b'), i.e., (meth)acrylamide or a (meth)acrylamide derivative, which is used at the time of polymerization of the copolymer (P).

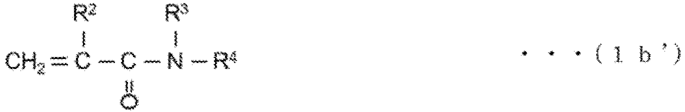

In the formula (1b'), R², R³, and R⁴ are the same as R², R³, and R⁴ in the formula (1b), respectively.

Examples of the (meth)acrylamide or the (meth)acrylamide derivative represented by the formula (1b') include N,N-dimethyl(meth)acrylamide, N,N-diethyl(meth)acrylamide, and N-acryloylmorpholine.

### <(3) Hydrophobic Constituent Unit>

The copolymer (P) to be used in the ophthalmic solution of the present invention has a constituent unit represented by the following formula (1c) (hereinafter abbreviated as "hydrophobic constituent unit").

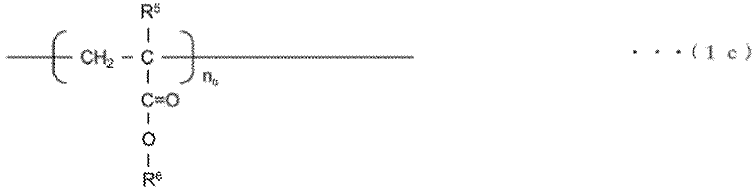

In the formula (1c), R⁵ represents a hydrogen atom or a methyl group, and R⁶ represents a monovalent hydrocarbon group having 12 to 24 carbon atoms, for example, a lauryl group, a stearyl group, or a behenyl group.

The hydrophobic constituent unit in the copolymer (P) is introduced in order to enhance a property of adsorbing onto a soft contact lens, enhance an ability to form a physically crosslinked gel through a hydrophobic interaction, and improve lubricity.

With regard to the ratio of the hydrophobic constituent unit in the copolymer (P), the ratio of the number of moles n_{c} to the number of moles nₐ of the PC constituent unit being defined as 100, i.e., n_{c}/nₐ is from 2/100 to 50/100, preferably from 5/100 to 25/100. When n_{c} is excessively small, the lubricity effect is not sufficiently sustained. When n_{c} is excessively large, the hydrophilicity of the copolymer (P) lowers to lower its solubility in an aqueous solution, resulting in a difficulty in producing the ophthalmic solution.

The hydrophobic constituent unit in the copolymer (P) is obtained from a hydrophobic monomer represented by the following formula (1c') that is used at the time of polymerization of the copolymer (P).

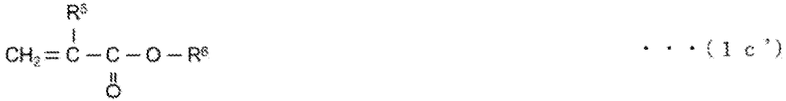

In the formula (1c'), R⁵ and R⁶ are the same as R⁵ and R⁶ in the formula (1c), respectively.

Examples of the hydrophobic monomer represented by the formula (1c') include linear alkyl (meth)acrylates, such as lauryl (meth)acrylate, stearyl (meth)acrylate, and behenyl (meth)acrylate.

### <Other Constituent Unit>

A constituent unit other than the constituent units represented by the formula (1a) to the formula (1c) may also be introduced into the copolymer (P) as long as the effects of the present invention are not impaired. When the other polymerizable monomer is blended into the monomer composition to be used in the production of the copolymer (P), the blending ratio of the other polymerizable monomer may be appropriately selected within a range in which the effects of the present invention are not affected. However, the blending ratio is preferably 50 or less in terms of molar ratio when nₐ of the constituent unit represented by the formula (1a) in the copolymer (P) is defined as 100.

Examples of the other polymerizable monomer that may be used for polymerization of the copolymer (P) may include a linear or branched alkyl (meth)acrylate, a cyclic alkyl (meth)acrylate, an aromatic group-containing (meth)acrylate, a styrene-based monomer, a vinyl ether monomer, a vinyl ester monomer, a hydrophilic hydroxy group-containing (meth) acrylate, an acid group-containing monomer, an amino group-containing monomer, a cationic group-containing monomer, and a nitrogen-containing group-containing monomer.

Examples of the linear or branched alkyl (meth) acrylate include methyl (meth)acrylate, ethyl (meth)acrylate, butyl (meth)acrylate, and 2-ethylhexyl (meth)acrylate.

An example of the cyclic alkyl (meth)acrylate is cyclohexyl (meth)acrylate.

Examples of the aromatic group-containing (meth)acrylate include benzyl (meth)acrylate and phenoxyethyl (meth)acrylate.

Examples of the styrene-based monomer include styrene, methylstyrene, and chloromethylstyrene.

Examples of the vinyl ether monomer include methyl vinyl ether and butyl vinyl ether.

Examples of the vinyl ester monomer include vinyl acetate and vinyl propionate.

Examples of the hydrophilic hydroxy group-containing (meth)acrylate include polyethylene glycol (meth)acrylate, polypropylene glycol (meth)acrylate, 2-hydroxyethyl (meth)acrylate, 2-hydroxybutyl (meth)acrylate, and 4-hydroxybutyl (meth)acrylate.

Examples of the acid group-containing monomer include (meth)acrylic acid, styrenesulfonic acid, and (meth)acryloyloxy phosphonic acid.

Examples of the amino group-containing monomer include aminoethyl methacrylate, dimethylaminoethyl (meth)acrylate, and N,N-dimethylaminopropyl (meth)acrylamide.

An example of the cationic group-containing monomer is 2-hydroxy-3-(meth)acryloyloxypropyltrimethylammonium chloride.

An example of the nitrogen-containing group-containing monomer is N-vinylpyrrolidone.

<Combination of PC Constituent Unit, Amide Constituent Unit, and Hydrophobic Constituent Unit>

Preferred combinations of the PC constituent unit, the amide constituent unit, and the hydrophobic constituent unit of the copolymer (P) to be used in the present invention are as described below, but are not particularly limited to the following:
a copolymer (P) in which the PC constituent unit is a constituent unit obtained from 2-(methacryloyloxy)ethyl-2'-(trimethyammonio)ethyl phosphate, the amide constituent unit is a constituent unit obtained from N,N-dimethyl(meth)acrylamide, and the hydrophobic constituent unit is a constituent unit obtained from stearyl (meth)acrylate; and
a copolymer (P) in which the PC constituent unit is a constituent unit obtained from 2-(methacryloyloxy)ethyl-2'-(trimethylammonio)ethyl phosphate, the amide constituent unit is a constituent unit obtained from N,N-dimethyl(meth)acrylamide, and the hydrophobic constituent unit is a constituent unit obtained from lauryl (meth)acrylate.

Further, the ratio (molar ratio nₐ:n_{b}:n_{c}) of the constituent units that are the PC constituent unit, the amide constituent unit, and the hydrophobic constituent unit in each of those copolymers (P) is 100:from 10 to 400:from 2 to 50. More specifically, n_{b}/nₐ is from 10/100 to 400/100, preferably from 30/100 to 250/100, and n_{c}/nₐ is from 2/100 to 50/100, preferably from 5/100 to 25/100.

### <Molecular Weight of Copolymer (P)>

The copolymer (P) to be used in the present invention is a polymer having a weight-average molecular weight of from 5,000 to 2,000,000, preferably from 100,000 to 1,500,000. When the weight-average molecular weight is less than 5,000, there is a risk in that the adsorption power of the polymer onto a contact lens surface is not sufficient and hence lubricity improvement cannot be expected. When the weight-average molecular weight is more than 2,000,000, there is a risk in that aseptic filtration to be required in the production of the ophthalmic solution may be difficult.

### <Production Method for Copolymer (P)>

The copolymer (P) may be obtained by radically polymerizing a blend of the above-mentioned monomers. The production of the copolymer (P) may be performed by, for example, radically polymerizing the monomer composition in the presence of a radical polymerization initiator under purging with or an atmosphere of an inert gas, for example, nitrogen, carbon dioxide, argon, or helium. The polymerization may be performed by a known method, for example, bulk polymerization, suspension polymerization, emulsion polymerization, or solution polymerization. The polymerization method is preferably solution polymerization from the viewpoint of, for example, purification. The copolymer (P) may be purified by a known purification method, for example, a reprecipitation method, a dialysis method, or an ultrafiltration method.

Examples of the radical polymerization initiator may include an azo-based radical polymerization initiator, an organic peroxide, and a persulfate.

Examples of the azo-based radical polymerization initiator include 2,2-azobis(2-diaminopropyl) dihydrochloride, 2,2-azobis(2-(5-methyl-2-imidazolin-2-yl)propane) dihydrochloride, 4,4-azobis(4-cyanovaleric acid), 2,2-azobisisobutylamide dihydrate, 2,2-azobis(2,4-dimethylvaleronitrile), and 2,2-azobisisobutyronitrile (AIBN).

Examples of the organic peroxide include t-butyl peroxyneodecanoate, benzoyl peroxide, diisopropyl peroxydicarbonate, t-butyl peroxy-2-ethylhexanoate, t-butyl peroxypivalate, t-butyl peroxydiisobutyrate, lauroyl peroxide, t-butyl peroxyneodecanate, and succinic acid peroxide (=succinyl peroxide).

Examples of the persulfate include ammonium persulfate, potassium persulfate, and sodium persulfate.

Those radical polymerization initiators may be used alone or as a mixture thereof. The polymerization initiator is generally used in an amount of from 0.001 part by mass to 10 parts by mass, preferably from 0.01 part by mass to 5.0 parts by mass with respect to 100 parts by mass of the monomer composition.

The copolymer (P) may be produced in the presence of a solvent. The solvent may be any solvent that is capable of dissolving the monomer composition and does not react with the composition. Examples thereof may include water, an alcohol-based solvent, a ketone-based solvent, an ester-based solvent, a linear or cyclic ether-based solvent, and a nitrogen-containing solvent.

Examples of the alcohol-based solvent include methanol, ethanol, n-propanol, and isopropanol.

Examples of the ketone-based solvent include acetone, methyl ethyl ketone, and diethyl ketone.

An example of the ester-based solvent is ethyl acetate.

Examples of the linear or cyclic ether-based solvent include ethyl cellosolve and tetrahydrofuran.

Examples of the nitrogen-containing solvent include acetonitrile, nitromethane, and N-methylpyrrolidone.

There is preferably given water or an alcohol-based solvent, or a mixed solvent thereof.

The ophthalmic solution of the present invention is obtained by dissolving the copolymer (P) in water at from 0.01 W/V% to 2.0 W/V%. When the concentration of the copolymer (P) is less than 0.01 W/V%, the lubricity-improving effect is not sufficient. When the concentration is more than 2.0 W/V%, there is a risk in that aseptic filtration to be performed in the production of the ophthalmic solution may be difficult.

In addition, the concentration of the copolymer (P) in the ophthalmic solution of the present invention is more preferably from 0.1 W/V% to 2.0 W/V% or from 0.01 W/V% to 1.5 W/V%, still more preferably from 0.1 W/V% to 1.5 W/V%.

As a component of the ophthalmic solution of the present invention, besides the copolymer (P), any other component, or a component to be generally used for an ophthalmic solution may be blended in an appropriate amount as appropriate in accordance with the purpose or the like as long as the effects of the present invention are not impaired and in expectation of other effects.

Examples of the other component may include a decongestant component, an anti-inflammation and astringent component, a vitamin, an amino acid, a sulfa drug, a saccharide, a viscosifying agent, a cooling agent, an inorganic salt, an organic acid salt, an acid, a base, an antioxidant, a stabilizer, an antiseptic, a mucopolysaccharide, and a mucin secretagogue.

Examples of the decongestant component include epinephrine or salts thereof, ephedrine hydrochloride, tetrahydrozoline hydrochloride, naphazoline or salts thereof, phenylephrine, and methylephedrine hydrochloride.

Examples of the anti-inflammation and astringent component include ε-aminocaproic acid, allantoin, berberine or salts thereof, sodium azulene sulfonate, glycyrrhizic acid or salts thereof, zinc lactate, zinc sulfate, and lysozyme chloride.

Examples of the vitamin include sodium flavin adenine dinucleotide, cyanocobalamin, retinol acetate, retinol palmitate, pyridoxine hydrochloride, panthenol, sodium pantothenate, and calcium pantothenate.

Examples of the amino acid include aspartic acid or salts thereof, and aminoethylsulfonic acid.

Examples of the sulfa drug include sulfamethoxazole or salts thereof, sulfisoxazole, and sodium sulfisomidine.

Examples of the saccharide include glucose, mannitol, sorbitol, xylitol, and trehalose.

An example of the viscosifying agent is hydroxypropyl methylcellulose.

Examples of the cooling agent include menthol and camphor.

Examples of the inorganic salt include sodium chloride, potassium chloride, borax, sodium hydrogen carbonate, sodium hydrogen phosphate, and anhydrous sodium dihydrogen phosphate.

An example of the organic acid salt is sodium citrate.

Examples of the acid include boric acid, phosphoric acid, citric acid, sulfuric acid, acetic acid, and hydrochloric acid.

Examples of the base include sodium hydroxide, potassium hydroxide, trishydroxymethylaminomethane, and monoethanolamine.

Examples of the antioxidant include tocopherol acetate and dibutylhydroxytoluene.

Examples of the stabilizer include sodium edetate and glycine .

Examples of the antiseptic include benzalkonium chloride, chlorhexidine gluconate, potassium sorbate, and polyhexanide hydrochloride.

Examples of the mucopolysaccharide include sodium hyaluronate and chondroitin sulfate sodium.

Examples of the mucin secretagogue include diquafosol sodium and rebamipide.

The ophthalmic solution of the present invention has the form of an aqueous solution in which the copolymer (P), and as desired, the above-mentioned other components are dissolved in water. The water is preferably pure water, ion-exchanged water, or the like from the standpoint of safety.

Specific product forms of the ophthalmic solution of the present invention may be exemplified by the following: general eye drops, antibiotic eye drops, eyewashes, contact lens wetting solutions, artificial tears, and the like.

Soft contact lenses are classified into the following four groups according to the Food and Drug Administration (FDA): Group I (soft contact lenses each having a water content of less than 50% and being nonionic), Group II (soft contact lenses each having a water content of 50% or more and being nonionic), Group III (soft contact lenses each having a water content of less than 50% and being ionic), and Group IV (soft contact lenses each having a water content of 50% or more and being ionic).

The ophthalmic solution of the present invention may be used for all those soft contact lenses. However, from the viewpoint of a lubricity-improving effect on the soft contact lens surface, the ophthalmic solution of the present invention is preferably used for soft contact lenses of Group IV.

A production method for the ophthalmic solution of the present invention is described.

The ophthalmic solution of the present invention may be produced by adding the copolymer (P), and as desired, the above-mentioned other components into water at from about room temperature to about 50°C, and stirring the mixture to dissolution. In addition, the order in which the copolymer (P) and the above-mentioned other components are added is not limited as to which component is added first.

With regard to heating, cooling, and stirring in the production, it is only necessary that the entire solution can be uniformly heated, cooled, and stirred. The heating, the cooling, and the stirring may each be performed by using a known instrument or apparatus.

The production of an ophthalmic solution, includes using a copolymer (P) having constituent units represented by the formula (1a) to the formula (1c), a ratio nₐ:n_{b}:n_{c} of the constituent units of 100:from 10 to 400:from 2 to 50, and a weight-average molecular weight of from 5,000 to 2,000,000: 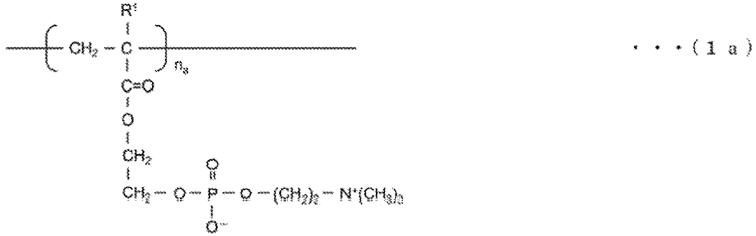 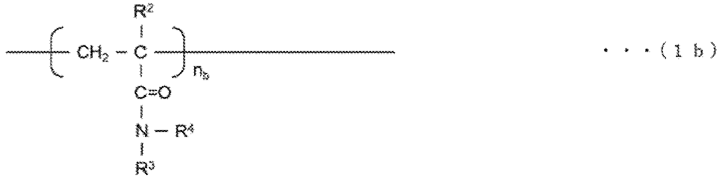 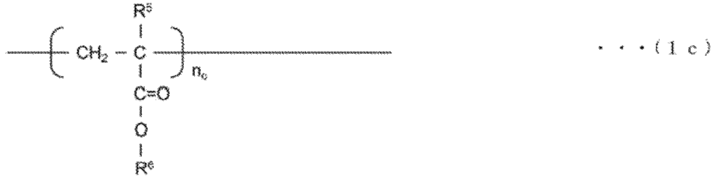 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.

An ophthalmic solution administration method of the present invention is described but is not part of the claims.

The ophthalmic solution administration method of the present invention for use in a method of treatment of a subject contains 0.01 W/V% to 2.0 W/V% of a copolymer (P) having constituent units represented by the formula (1a) to the formula (1c), a ratio nₐ:n_{b}:n_{c} of the constituent units of 100:from 10 to 400:from 2 to 50, and a weight-average molecular weight of from 5,000 to 2,000,000: 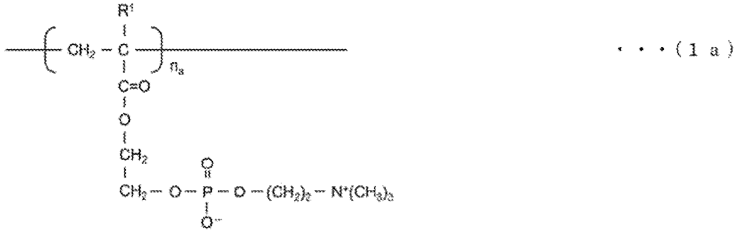 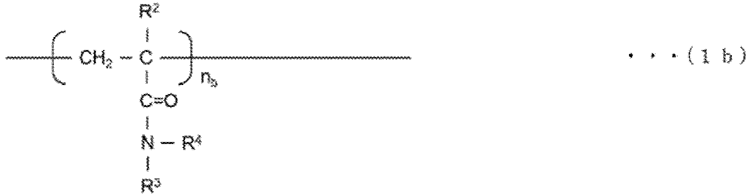 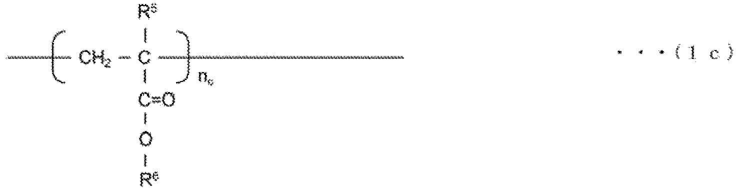 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.

The ophthalmic solution method of treatment (dropping method) of the present invention is not particularly limited, and the ophthalmic solution of the present invention may be dropped at a single dose of from 0.01 ml to 0.2 ml to an eye (eyeball) from any angle 1 to 10 times, 1 to 8 times, 1 to 6 times, 1 to 4 times, or 1 to 3 times a day (preferably in the morning, the afternoon, and the evening).

A target of the ophthalmic solution administration method is, a soft contact lens wearer. In addition, the target is a patient in need of treatment or prevention of lid wiper epitheliopathy.

### Examples

Now, the present invention and the effects thereof are specifically described by way of Examples and Comparative Examples of the present invention.

### 1. Measurement of Molecular Weight of Polymer

5 mg of each obtained polymer was dissolved in a methanol/chloroform mixed liquid (80:20) to prepare a sample solution. The following analysis conditions were used.
Column: PLgel MIXED-C
Standard substance: polyethylene glycol
Detector:differentialrefractometerRI-8020(manufacturedby Tosoh Corporation)
Calculation method for weight-average molecular weight: molecular weight calculation program (GPC program for SC-8020)
Flow rate: 1 mL/min
Injection amount: 100 µL
Column oven: constant temperature around 40°C

The weight-average molecular weight of the polymer is a value of a weight-average molecular weight measured with a gel permeation chromatograph (GPC) using polyethylene glycol as a standard sample.

The resultant polymer solution was diluted to 0.5 mass% with water, and the resultant liquid was filtered through a 0.45 µm membrane filter before measurement.

### 2. pH of Ophthalmic Solution

The pH of an ophthalmic solution of each of Examples and Comparative Examples was measured in accordance with "the Japanese Pharmacopoeia, Sixteenth Edition, General Tests, Processes and Apparatus, 2.54 pH Determination."

### 3. Osmotic Pressure of Ophthalmic Solution

The osmotic pressure of the ophthalmic solution of each of Examples and Comparative Examples was measured in accordance with "the Japanese Pharmacopoeia, Sixteenth Edition, General Tests, Processes and Apparatus, 2.47 Osmolarity Determination." Specifically, measurement was performed with an osmometer (Fiske Model 210 Micro-Sample Osmometer) based on a freezing-point measurement method.

### [Synthesis Example 1]

31.8 g of MPC (manufacturedby NOF Corporation), 3.6 g of stearyl methacrylate (SMA, manufactured by NOF Corporation), and 9.6 g of N,N-dimethylacrylamide (DMAA, manufactured by KOHJIN Film & Chemicals Co., Ltd.) were placed in a four-necked flask, and dissolved with 55.0 g of ethanol, followed by nitrogen gas blowing for 30 minutes. After that, 0.10 g of t-butylperoxyneodecanoate (PERBUTYL (registered trademark in Japan) ND (PB-ND), manufactured by NOF Corporation) was added as a polymerization initiator, and the mixture was subjected to a polymerization reaction for 8 hours. After the polymerization reaction, the polymerization liquid was added dropwise into 3 L of diethyl ether under stirring, and the resultant precipitate was filtered and vacuum-dried at room temperature for 48 hours to provide powder. The yield was 40.2 g. Molecular weight analysis of the polymerization product was performed by GPC, and its weight-average molecular weight was found to be 1,000,000. The polymerization product was defined as a polymer 1. Analysis results of IR, NMR, and elemental analysis are shown below.

IR analysis results: 2, 964 cm⁻¹ (-CH), 1, 733 cm⁻¹ (O-C=O), 1,651 cm⁻¹ (N-C=O), 1,458 cm⁻¹ (-CH), 1,253 cm⁻¹ (P=O), 1,168 cm⁻¹ (C-O-C), and 997 cm⁻¹(P-O-C).

NMR analysis results: 0.8-1.2 ppm (CH₃-C-), 1.4 ppm (-CH₂-), 3.3 ppm (-N(CH₃)₃), 2.8-3.2 ppm (-N-(CH₃)₂), and 3.7-4.4 ppm (-CH₂CH₂-).

Element analysis results:
Theoretical value: C; 53.55%, H; 8.44%, N; 8.74%
Actual measured value: C; 53.40%, H; 8.52%, N; 8.80%

The above-mentioned results revealed that the obtained polymer 1 was, in terms of chemical structure, a polymer in which MPC, DMAA, and SMA were copolymerized at ratios of 50 mol%, 45 mol%, and 5 mol%, respectively.

### [Synthesis Example 2]

A polymer was produced in accordance with the same procedure as that of Synthesis Example 1 except that components whose kinds and amounts were as shown in Table 1 below were used. The weight-average molecular weight of the polymer was 1,200,000, and its yield was 42.4 g. Analysis results of IR, NMR, and elemental analysis are shown below.

IR analysis results: 2, 964 cm⁻¹ (-CH), 1, 733 cm⁻¹ (O-C=O), 1,651 cm⁻¹ (N-C=O), 1,458 cm⁻¹ (-CH), 1,253 cm⁻¹ (P=O), 1,168 cm⁻¹ (C-O-C), and 997 cm⁻¹ (P-O-C).

NMR analysis results: 0.8-1.2 ppm (CH₃-C-), 1.4 ppm (-CH₂-), 3.3 ppm (-N(CH₃)₃), 2.8-3.2 ppm (-N-(CH₃)₂) and 3.7-4.4 ppm (-CH₂CH₂-).

Element analysis results:
Theoretical value: C; 56.37%, H; 8.70%, N; 10.90%
Actual measured value: C; 56.41%, H; 8.69%, N; 10.87%

The above-mentioned results revealed that the obtained polymer 2 was, in terms of chemical structure, a polymer in which MPC, DMAA, and SMA were copolymerized at ratios of 30 mol%, 67 mol%, and 3 mol%, respectively.

### [Synthesis Example 3]

A polymer was produced in accordance with the same procedure as that of Synthesis Example 1 except that components whose kinds and amounts were as shown in Table 1 below were used. The weight-average molecular weight of the polymer was 700,000, and its yield was 36.1 g. Analysis results of IR, NMR, and elemental analysis are shown below.

IR analysis results: 2, 964 cm⁻¹ (-CH), 1,733 cm⁻¹ (O-C=O), 1,651 cm⁻¹ (N-C=O), 1,458 cm⁻¹ (-CH), 1,253 cm⁻¹ (P=O), 1,168 cm⁻¹ (C-O-C), and 997 cm⁻¹ (P-O-C).

NMR analysis results: 0.8-1.2 ppm (CH₃-C-), 1.4 ppm (-CH₂-), 3.3 ppm (-N(CH₃)₃), 2.8-3.2 ppm (-N-(CH₃)₂) and 3.7-4.4 ppm (-CH₂CH₂-).

Element analysis results:
Theoretical value: C; 50.55%, H; 8.15%, N; 6.72%
Actual measured value: C; 50.46%, H; 8.14%, N; 6.72%

The above-mentioned results revealed that the obtained polymer 3 was, in terms of chemical structure, a polymer in which MPC, DMAA, and SMA were copolymerized at ratios of 70 mol%, 24 mol%, and 6 mol%, respectively.

### [Synthesis Example 4]

A polymer was produced in accordance with the same procedure as that of Synthesis Example 1 except that components whose kinds and amounts were as shown in Table 1 below were used. The weight-average molecular weight of the polymer was 1,000,000, and its yield was 39.9 g. Analysis results of IR, NMR, and elemental analysis are shown below.

IR analysis results: 2, 964 cm⁻¹ (-CH), 1, 733 cm⁻¹ (O-C=O), 1,651 cm⁻¹ (N-C=O), 1,458 cm⁻¹ (-CH), 1,253 cm⁻¹ (P=O), 1,168 cm⁻¹ (C-O-C), and 997 cm⁻¹ (P-O-C).

NMR analysis results: 0.8-1.2 ppm (CH₃-C-), 1.4 ppm (-CH₂-), 3.3 ppm (-N(CH₃)₃), 2.8-3.2 ppm (-N-(CH₃)₂) and 3.7-4.4 ppm (-CH₂CH₂-).

Element analysis results:
Theoretical value: C; 54.17%, H; 8.55%, N; 8.03%
Actual measured value: C; 54.12%, H; 8.54%, N; 8.04%

The above-mentioned results revealed that the obtained polymer 4 was, in terms of chemical structure, a polymer in which MPC, DMAA, and lauryl methacrylate (LMA) were copolymerized at ratios of 50 mol%, 40 mol%, and 10 mol%, respectively.

**Table 1**

| | Synthesis Example 1 | | Synthesis Example 2 | | Synthesis Example 3 | | Synthesis Example 4 | |
|---|---|---|---|---|---|---|---|---|
| | Polymer 1 | | Polymer 2 | | Polymer 3 | | Polymer 4 | |
| | mol% | Weight (g) | mol% | Weight (g) | mol% | Weight (g) | mol% | Weight (g) |
| MPC (formula (1a)) | 50 | 31.8 | 30 | 24.1 | 70 | 33 | 50 | 31.2 |
| DMAA (formula (1b)) | 45 | 9.6 | 67 | 18.1 | 24 | 3.8 | 40 | 8.4 |
| LMA (formula (1c)) | | | | | | | 10 | 5.4 |
| SMA (formula (1d)) | 5 | 3.6 | 3 | 2.8 | 6 | 3.2 | | |
| Total of monomers (g) | 45 | | 45 | | 40 | | 45 | |
| Ethanol (g) | 55 | | | | | | 55 | |
| n-Propanol (g) | | | 55 | | 60 | | | |
| PB-ND (g) | 0.1 | | 0.1 | | 0.1 | | 0.1 | |
| Weight-average molecular weight | 1,000,000 | | 1,200,000 | | 700,000 | | 1,000,000 | |
| Yield (g) | 40.2 | | 42.4 | | 36.1 | | 39.9 | |

### [Example 1]

About 80 g of purified water was warmed to 45°C. To the warmed water, 0.4 g of boric acid, 0.0072 g of sodium hydroxide, 0.55 g of sodium chloride, 0.1 g of potassium chloride, 0.0004 g of Cosmocil CQ (registered trademark in Japan (trade mark)) (which was a 20% aqueous solution, and hence contained 0.00008 g of polyhexanide hydrochloride), and 0.1 g of the polymer 1 were sequentially added, and the mixture was stirred. The solution was stirred and mixed for 1 hour while being kept at 45°C. After that, purified water was added thereto to a total volume of 100 mL. After that, the mixture was subjected to filter sterilization to obtain a sterile ophthalmic solution. The ophthalmic solution had an osmotic pressure of 281 mOsm/kg, a pH of 7.4, and a colorless and clear external appearance. The details thereof are shown in Table 2 below.

### [Example 2 to Example 6]

Sterile ophthalmic solutions were produced in accordance with the same procedure as that of Example 1 except that components whose kinds and amounts were as shown in Table 2 were used. The external appearance, pH, and osmotic pressure of each of Examples are shown in Table 2 below.

**Table 2**

| | | Example 1 | Example 2 | Example 3 | Example 4 | Example 5 | Example 6 |
|---|---|---|---|---|---|---|---|
| Polymer component | Polymer 1 | 0.1 | | | | | |
| | Polymer 2 | | 0.1 | | | | |
| | Polymer 3 | | | 0.1 | | | |
| | Polymer 4 | | | | 0.01 | 0.1 | 2 |
| Other component s | Sodium chloride | 0.55 | 0.55 | 0.55 | 0.7 | 0.7 | 0.7 |
| | Potassium chloride | 0.1 | 0.1 | 0.1 | 0.1 | 0.1 | 0.1 |
| | Sodium hydrogen phosphate hydrate | | | | 0.43 | 0.43 | 0.43 |
| | Anhydrous sodium dihydrogen phosphate | | | | 0.032 | 0.032 | 0.032 |
| | Boric acid | 0.4 | 0.4 | 0.4 | | | |
| | Sodium hydroxide | 0.0072 | 0.0072 | 0.0072 | | | |
| | Benzalkonium chloride | | | | 0.005 | | |
| | Chlorhexidine gluconate | | | | | 0.002 | |
| | Potassium sorbate | | | | | | 0.1 |
| | Polyhexanide hydrochloride | 0.00008 | 0.00008 | 0.00008 | | | |
| | Purified water | Total volume is adjusted to 100 mL | Total volume is adjusted to 100 mL | Total volume is adjusted to 100 mL | Total volume is adjusted to 100 mL | Total volume is adjusted to 100 mL | Total volume is adjusted to 100 mL |
| Analysis result | External appearance | Colorless and clear | Colorless and clear | Colorless and clear | Colorless and clear | Colorless and clear | Colorless and clear |
| | pH | 7.4 | 7.4 | 7.4 | 7.4 | 7.4 | 7.4 |
| | Osmotic pressure | 281 | 280 | 282 | 284 | 284 | 283 |

### [Comparative Example 1 to Comparative Example 5]

Sterile ophthalmic solutions were produced in accordance with the same procedure as those of Examples except that components whose kinds and amounts were as shown in Table 3 below were used. The external appearance, pH, and osmotic pressure of each of Comparative Examples are shown in Table 3 below.

**Table 3**

| | | Comparative Example 1 | Comparative Example 2 | Comparative Example 3 | Comparative Example 4 | Comparative Example 5 |
|---|---|---|---|---|---|---|
| Polymer component | Polymer 1 | 0.005 | | | | |
| | Polymer 2 | | 0.005 | | | |
| | Polymer 3 | | | 0.005 | | |
| | Polymer 4 | | | | 0.005 | |
| Other component s | Sodium chloride | 0.55 | 0.55 | 0.55 | 0.7 | 0.7 |
| | Potassium chloride | 0.1 | 0.1 | 0.1 | 0.1 | 0.1 |
| | Sodium hydrogen phosphate hydrate | | | | 0.43 | 0.43 |
| | Anhydrous sodium dihydrogen phosphate | | | | 0.032 | 0.032 |
| | Boric acid | 0.4 | 0.4 | 0.4 | | |
| | Sodium hydroxide | 0.0072 | 0.0072 | 0.0072 | | |
| | Benzalkonium chloride | | | | 0.005 | |
| | Chlorhexidine gluconate | | | | | 0.002 |
| | Potassium sorbate | | | | | |
| | Polyhexanide hydrochloride | 0.00008 | 0.00008 | 0.00008 | | |
| | Purified water | Total volume is adjusted to 100 mL | Total volume is adjusted to 100 mL | Total volume is adjusted to 100 mL | Total volume is adjusted to 100 mL | Total volume is adjusted to 100 mL |
| Analysis result | External appearance | Colorless and clear | Colorless and clear | Colorless and clear | Colorless and clear | Colorless and clear |
| | pH | 7.4 | 7.4 | 7.4 | 7.4 | 7.4 |
| | Osmotic pressure | 281 | 282 | 282 | 284 | 283 |

### <Investigation of Lubricity-improving Effect on SCL>

According to the literature "D.R. Korb et al., 2005, Eye & Contact Lens, 31, 2-8," it may be effective to reduce friction on a corneal surface for treatment of eyelid conjunctival epitheliopathy in some cases. However, a friction reduction effect on the corneal surface is difficult to verify. Therefore, the following procedure was employed to investigate a lubricity-improving effect at the time of the use of a soft contact lens assuming actual use.

For the investigation of the lubricity-improving effect on the SCL, 1-Day ACUVUE (registered trademark in Japan (trade mark)) (manufactured by Johnson & Johnson, FDA classification: Group IV) and Medalist Plus (registered trademark in Japan (trade mark)) (manufactured by B.L.J. Company, Ltd., FDA classification: Group I) were used.

### (Procedure)

1) The ophthalmic solution of Example 1 was diluted 10-fold with saline.
2) One test soft contact lens was taken out of a blister pack, and placed in a 15 mL centrifuge tube.
3) 10 mL of saline was added thereto, and the whole was shaken overnight.
4) After that, the saline was removed, and 10 mL of the solution prepared in 1) was added, followed by overnight shaking.
5) After the shaking, the soft contact lens was removed, and placed on an index finger to perform lubricity evaluation.

The ophthalmic solutions of Example 2 to Example 6 and Comparative Example 1 to Comparative Example 5 were also evaluated in accordance with the above-mentioned procedure.

A method for the lubricity evaluation was as follows: with the use of 1-Day ACUVUE (registered trademark in Japan (trade mark)) that had just been taken out of a blister pack as a reference (4 points), an evaluation score was raised as the lubricity improved, and the evaluation score was lowered as the lubricity lowered. The evaluation score was given within the range of from 1 point to 10 points.

The results of the lubricity evaluation are shown in Table 4 below. In the case of 1-Day ACUVUE (registered trademark in Japan (trade mark)), in Example 4, the evaluation score was 6 points, i.e., an improvement in lubricity was observed, and moreover, in each of Example 1 to Example 3, Example 5, and Example 6, the evaluation score was 10 points, i.e., the best evaluation result was obtained. In each of Comparative Example 1 to Comparative Example 5, the result was below the reference of 4 points, i.e., a result of lowering lubricity was obtained.

In the case of Medalist Plus (registered trademark in Japan (trade mark)), in Example 4, the evaluation score was 5 points, and in each of Examples 1 to 3, Example 5, and Example 6, the evaluation score was 9 points. In each of Comparative Example 1 to Comparative Example 5, the result was below the reference of 4 points as in the case of 1-Day ACUVUE (registered trademark in Japan (trade mark)), i.e., a result of lowering lubricity was obtained.

Comparing the results for 1-Day ACUVUE (registered trademark in Japan (trade mark)) and Medalist Plus (registered trademark in Japan (trade mark)), it was found that 1-Day ACUVUE (registered trademark in Japan (trade mark)) had more improved lubricity.

The foregoing reveals that the ophthalmic solution of the present invention exhibits excellent lubricating performance.

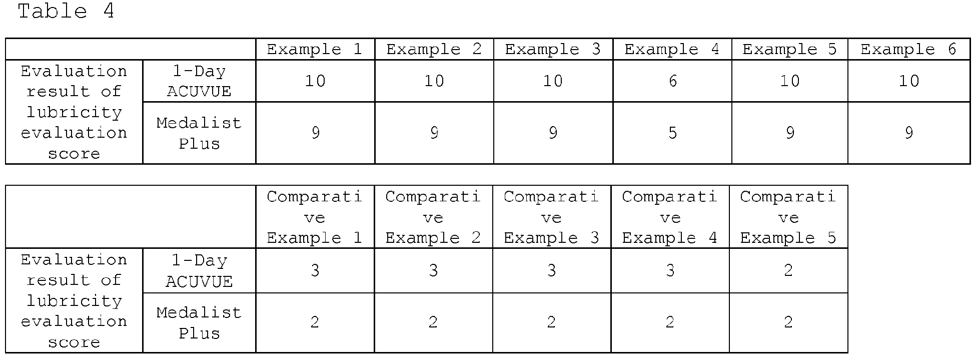

### <Investigation on Treatment and Prevention of Lid Wiper Epitheliopathy using Rabbit Dry Eye Model>

A treatment effect on eyelid conjunctival epitheliopathy was investigated. The investigation was performed in accordance with the following procedure with reference to the literature "T. Nagano et al., Atarashii Ganka (Journal of the Eye), 13, 267-270, 1996", the literature "A. Shiraishi et al., Nippon Ganka Gakkai Zasshi (J. Jpn. Ophthalmol Soc.), 113, 596-600, 2009.," and the literature "D.R. Korb et al., CLAO J, 28, 211-216, 2002."

### (Procedure)

1) An eyelid of a rabbit was forcibly retracted, and then a soft contact lens (1-Day ACUVUE (manufactured by Johnson & Johnson) (registered trademark in Japan (trade mark))) was inserted.
2) The soft contact lens was kept inserted for 16 hours to cause lid wiper epitheliopathy.
3) After that, the soft contact lens was removed, and epithelial tissues of the upper eyelid conjunctiva and the lower eyelid conjunctiva were stained with methylene blue, followed by observation of the degree of lid wiper epitheliopathy. The degree of epitheliopathy was scored on the basis of the staining.
   0 points: No abnormal finding
   1 point: Mild
   2 points: Moderate
   3 points: Severe

   For the test, rabbits each having an overall score for the upper and lower eyelids of 5 points or more were used.
4) The ophthalmic solution of Example 1 was administered to each of the rabbits each having a score of 5 points or more at a single dose of one drop 3 times a day for 3 days.
5) After the administration for 3 days, staining was performed again with methylene blue, and the degree of lid wiper epitheliopathy was observed.

Scoring was performed for both the upper eyelid conjunctiva and the lower eyelid conjunctiva, and evaluation was performed on the basis of the total score. The ophthalmic solutions of Example 2 to Example 6 and Comparative Example 1 to Comparative Example 5 were also evaluated in accordance with this procedure.

The evaluation results are shown in Table 5 below. In Example 4, the score after the test was 3 points, i.e., an improvement was shown, and in each of Example 1 to Example 3, Example 5, and Example 6, the score after the test was 1 point, i.e., a significant improving effect was shown. Meanwhile, in each of Comparative Example 1 to Comparative Example 4, an improving tendency was shown but the degree of improvement was small, and in Comparative Example 5, no improving tendency was shown.

The foregoing reveals that the ophthalmic solution and the ophthalmic solution method of treatment of the present invention exhibit excellent treatment and prevention effects on lid wiper epitheliopathy.

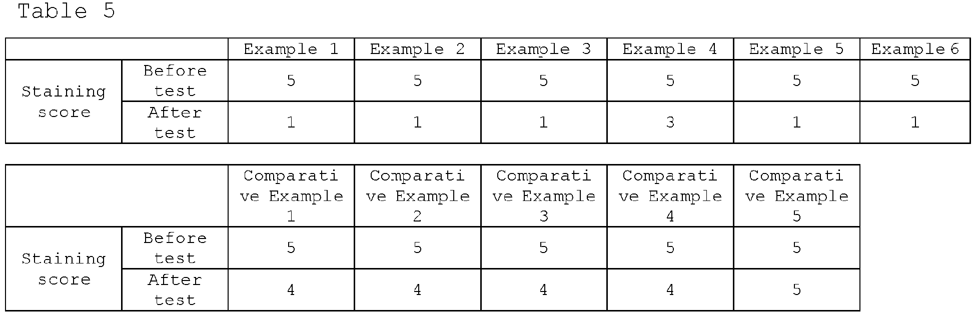

### Industrial Applicability

The present invention can provide the novel ophthalmic solution and ophthalmic solution method of treatment.

## Claims

1. An ophthalmic aqueous solution for use in treatment or prevention of lid wiper epitheliopathy, the solution comprising 0.01 W/V% to 2.0 W/V% of a copolymer (P) having constituent units represented by the formula (1a) to the formula (1c), a ratio nₐ:n_{b}:n_{c} of the constituent units of 100:from 10 to 400:from 2 to 50, and a weight-average molecular weight of from 5,000 to 2,000,000: 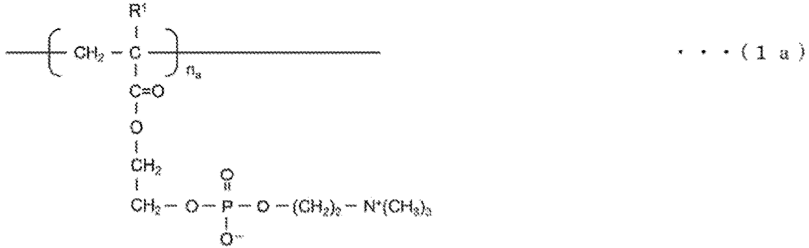 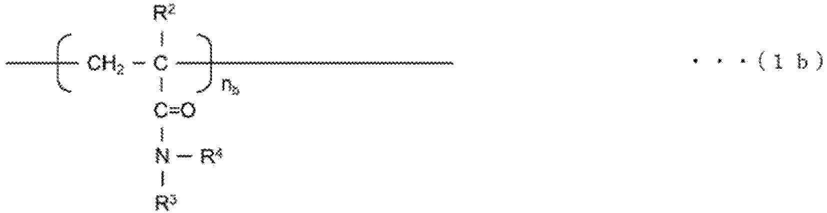 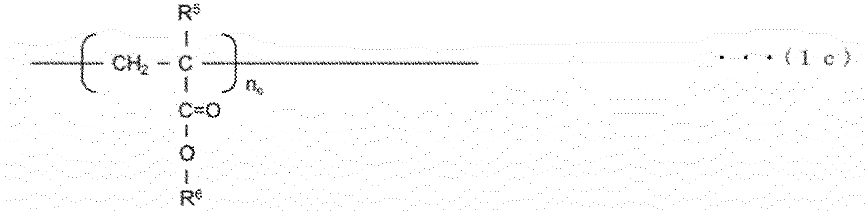 where R¹, R², and R⁵ each independently represent a hydrogen atom or a methyl group, R³ and R⁴ each independently represent a hydrogen atom, a methyl group, or an ethyl group, or are bonded to each other to represent a morpholino group, and R⁶ represents a monovalent hydrocarbon group having 12 to 24 carbon atoms.

2. An ophthalmic aqueous solution for use according to claim 1, wherein the (1a) comprises a constituent unit obtained from 2-(methacryloyloxy)ethyl-2'-(trimethylammonio)ethyl phosphate, the (1b) comprises a constituent unit obtained from N,N-dimethyl(meth)acrylamide, and the (1c) comprises a constituent unit obtained from stearyl (meth)acrylate.

3. An ophthalmic aqueous solution for use according to claim 1, wherein the (1a) comprises a constituent unit obtained from 2-(methacryloyloxy)ethyl-2'-(trimethylammonio)ethyl phosphate, the (1b) comprises a constituent unit obtained from N,N-dimethyl(meth)acrylamide, and the (1c) comprises a constituent unit obtained from lauryl (meth)acrylate.

4. An ophthalmic aqueous solution for use according to any one of claims 1 to 3, wherein the ophthalmic solution is for use in a method of treatment of a subject, wherein the subject is a soft contact lens wearer.

## Patentansprüche

1. Ophthalmische wässrige Lösung zur Verwendung zur Behandlung oder Prävention einer Lid-Wiper-Epitheliopathie, wobei die Lösung 0,01 Gew./Vol.-% bis 2,0 Gew./Vol.-% eines Copolymers (P) umfasst, das Grundeinheiten der Formeln (1a) bis (1c), ein Verhältnis nₐ:n_{b}:n_{c} der konstituierenden Einheiten von 100 : 10 bis 400 :2 bis 50 sowie ein gewichtsmittleres Molekulargewicht von 5.000 bis 2.000.000 aufweist: 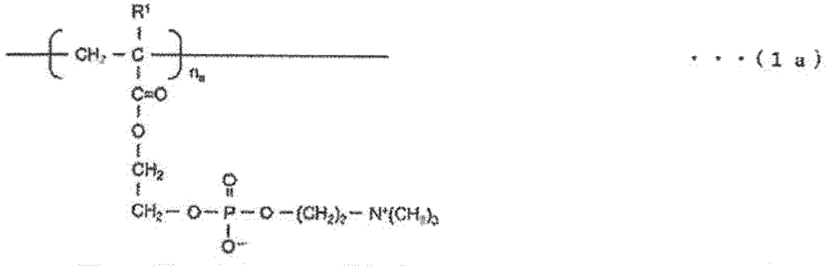 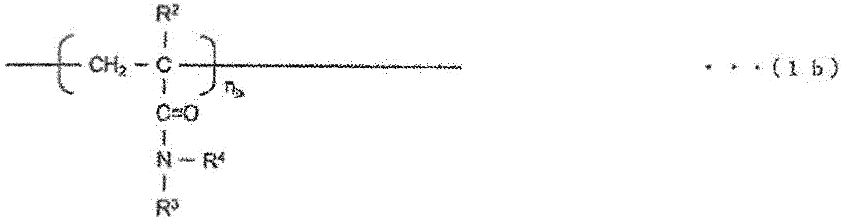 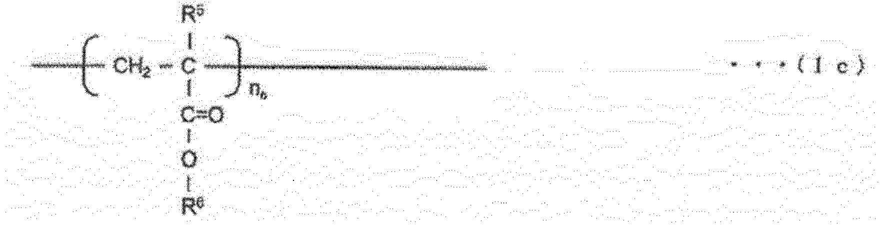 worin R¹, R² und R⁵ jeweils unabhängig für ein Wasserstoffatom oder eine Methylgruppe stehen, R³ und R⁴ jeweils unabhängig für ein Wasserstoffatom, eine Methylgruppe oder eine Ethylgruppe stehen oder miteinander zu einer Morpholinogruppe verbunden sind und R⁶ für eine einwertige Kohlenwasserstoffgruppe mit 12 bis 24 Kohlenstoffatomen steht.

2. Ophthalmische wässrige Lösung zur Verwendung nach Anspruch 1, wobei (1a) eine Grundeinheit, die aus 2-(Methacryloyloxy)ethyl-2'-(trimethylammonio)ethylphosphat erhalten wird, (1b) eine konstituierende Einheit, die aus N,N-Dimethyl(meth)acrylamid erhalten wird, und (1c) eine konstituierende Einheit, die aus Stearyl(meth)acrylat erhalten wird, umfasst.

3. Ophthalmische wässrige Lösung zur Verwendung nach Anspruch 1, wobei (1a) eine Grundeinheit, die aus 2-(Methacryloyloxy)ethyl-2'-(trimethylammonio)ethylphosphat erhalten wird, (1b) eine konstituierende Einheit, die aus N,N-Dimethyl(meth)acrylamid erhalten wird, und (1c) eine konstituierende Einheit, die aus Lauryl(meth)acrylat erhalten wird, umfasst.

4. Ophthalmische wässrige Lösung zur Verwendung nach einem der Ansprüche 1 bis 3, wobei die ophthalmische Lösung zur Verwendung in einem Verfahren zur Behandlung eines Individuums dient, wobei das Individuum ein Träger weicher Kontaktlinsen ist.

## Revendications

1. Solution ophtalmique aqueuse pour une utilisation dans le traitement ou la prévention d'une épithéliopathie de la conjonctive palpébrale, la solution comprenant 0,01 % p/v à 2,0 % p/v d'un copolymère (P) ayant des motifs constitutifs représentés par les formules (1a) à (1c), un rapport nₐ/n_{b}/n_{c} des motifs constitutifs de 100/10 à 400/2 à 50, et une masse moléculaire moyenne en masse de 5 000 à 2 000 000 : 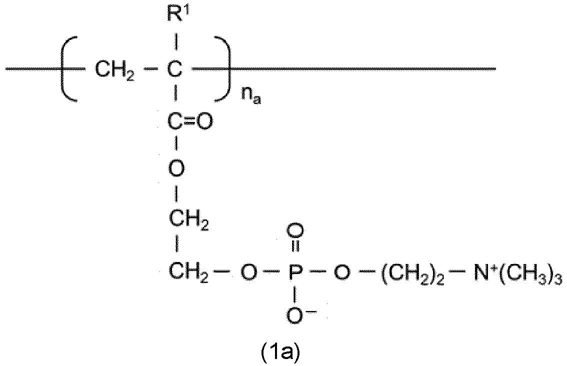 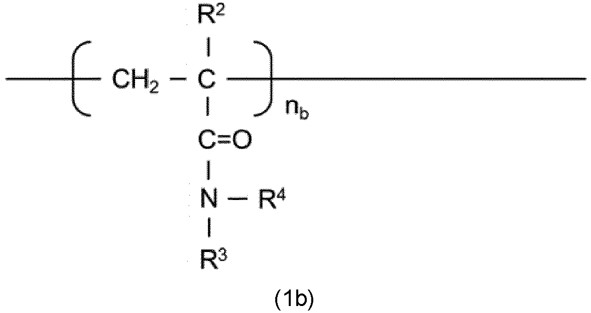 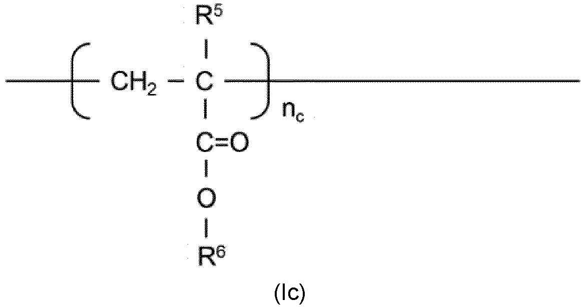 où chacun de R¹, R² et R⁵ représente indépendamment un atome d'hydrogène ou un groupe méthyle, chacun de R³ et R⁴ représente indépendamment un atome d'hydrogène, un groupe méthyle ou un groupe éthyle, ou sont liés l'un à l'autre pour représenter un groupe morpholino, et R⁶ représente un groupe hydrocarboné monovalent ayant 12 à 24 atomes de carbone.

2. Solution ophtalmique aqueuse pour une utilisation selon la revendication 1, dans laquelle (1a) comprend un motif constitutif obtenu à partir de phosphate de 2-(méthacryloyloxy)éthyl-2'-(triméthylammonio)éthyle, (1b) comprend un motif constitutif obtenu à partir de N,N-diméthyl(méth)acrylamide, et (1c) comprend un motif constitutif obtenu à partir de (méth)acrylate de stéaryle.

3. Solution ophtalmique aqueuse pour une utilisation selon la revendication 1, dans laquelle (1a) comprend un motif constitutif obtenu à partir de phosphate de 2-(méthacryloyloxy)éthyl-2'-(triméthylammonio)éthyle, (1b) comprend un motif constitutif obtenu à partir de N,N-diméthyl(méth)acrylamide, et (1c) comprend un motif constitutif obtenu à partir de (méth)acrylate de lauryle.

4. Solution ophtalmique aqueuse pour une utilisation selon l'une quelconque des revendications 1 à 3, laquelle solution ophtalmique est destinée à être utilisée dans une méthode de traitement d'un sujet, dans laquelle le sujet est un porteur de lentilles de contact souples.
